# EUROPEAN PATENT SPECIFICATION

(11) **EP 2 314 597 B1**
(45) Date of publication and mention of the grant of the patent: **28.11.2012**
(21) Application number: 10188077.1
(22) Date of filing: 19.10.2010
(51) Int. Cl.: C07K 1/13, G01N 33/533

(54) **Kit and method for the labelling of biomolecules**
Kit und Verfahren zur Markierung von Biomolekülen
Kit et procédé pour le marquage de biomolécules

(30) Priority: 21.10.2009 IT BO20090682
(43) Date of publication of application: 27.04.2011
(73) Proprietor: Cyanagen Srl, 40138 Bologna (IT)
(72) Inventor: Della Ciana, Leopoldo, 40138 Bologna (IT)
(74) Representative: Freyria Fava, Cristina

(56) References cited:
- EP-A1- 0 967 205
- WO-A2-2004/086050
- WO-A2-2008/108941
- US-A1- 2003 125 561
- US-A1- 2005 148 596
- BRUSCHI M ET AL: "New iodo-acetamido cyanines for labeling cysteine thiol residues. A strategy for evaluating plasma proteins and their oxido-redox status", PROTEOMICS, vol. 9, no. 2, 13 January 2009 (2009-01-13), pages 460-469, XP002586171, DOI: 10.1002/pmic.200800254

## Description

### FIELD OF THE INVENTION

The present disclosure relates to a kit and a method for the labelling of biomolecules.

### STATE OF THE ART

The labelling of biomolecules bearing amino groups is of great importance in many areas of biology and biotechnology, in particular in the fields of diagnostics, immunoassays, nucleic acid assays, bioimaging, and therapeutics.

For these purposes, the labelling compound is often an isothiocyanate, a sulfonyl chloride, or an active ester. Active esters are currently preferred, in particular N-hydrosuccinimide (NHS) esters or sulfo N-hydroxysuccinimide (sulfo-NHS) esters, see, e.g., Hermanson, "Bioconjugated Techniques", Academic Press (1996). This type of active esters is prepared from the corresponding carboxylic acid and N-hydroxysuccimides by means of carbodiimides, such as dicylohexylcarbodiimide (DCC) or N-(3-dimethylaminopropyl)-N'-ethylcarbodiimide (EDC), or carbonylimidazole. Alternatively, the carboxylic acid can be converted into the corresponding N-hydroxysuccinimide (NHS) or sulfo-NHS ester by means of disuccinimidylcarbonate. The active ester is isolated, generally as a solid, and stored under dry conditions.

In most cases, NHS and sulfo-NHS esters are very moisture sensitive and cannot be easily purified by crystallization or chromatography. Therefore, they often contain considerable and variable amounts of impurities, especially of the starting carboxylic acid. In some instances, isolation of the active ester produces a large increase in non-specific binding upon conjugation e.g., the NHS ester of the chemiluminescent label 7-(4-aminobutyl-N-ethyl)naphthalene-12-dicarboxylic acid hydrazide hemisuccinic acid(ABEN-H) Chapter 3, p. 146, in "Luminescence Immunoassays and Molecular Applications", Van Dyke and Van Dyke Eds. CRC Press (1990).

NHS and sulfo-NHS active esters of certain compounds, especially fluorescent dyes, such as cyanines, must be stored under very dry conditions. However, unlike their carboxylic acid precursors, such active esters are often sensitive to electrostatic charges, and therefore difficult to weigh. Aliquoting of such active esters, which are often very expensive to fabricate, is therefore cumbersome; in addition even their DMF or DMSO solutions are of limited stability, being sensitive to even traces of moisture.

From the above discussion, it appears that the current use of isolated active esters to label biomolecules is often unreliable and wasteful. Because both the active ester and the biomolecules to be labelled are usually very costly, these problems represent a serious economic loss.

Therefore, it would be desirable to have a method for the rapid, quantitative *in situ* activation of a moisture stable, purifiable carboxylated labelling precursor compound.

Bruschi et al., Proteomics 9: 460-468, 2009, disclose a method for labelling plasma proteins using iodoacetamide-substituted cyanines.

*U5-A-2003*/*125561 and WO-A-2008*/*10941 disclose the use of uronium and guanidinium salts, respectively, as coupling agents in the synthesis of peptides.*

### SUMMARY OF THE INVENTION

Taking into consideration these premises, it is therefore felt the need for ameliorative solutions, more effective for labelling biomolecules bearing reactive amino or hydroxyl groups.

According to the invention, the above object is achieved thanks to the solution as called for in the claims that follow, which are an integral part of the disclosure of the invention as provided herein.

An embodiment of the present invention concerns a kit for labelling biomolecules bearing reactive amino or hydroxyl groups comprising a first component (Reagent A) and a second component (Reagent B), wherein said first component comprises a mixture of a carboxylated labelling compound and a tertiary amine, and wherein said second component comprises:
i) a guanidinium salt of formula (a) 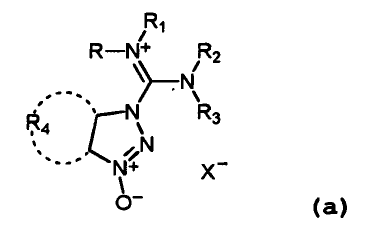 wherein
   R, R₁, R₂, R₃ are independently selected from hydrogen, substituted or unsubstituted alkyl having 1 to 6 carbon atoms, substituted or unsubstituted alkenyl having 2 to 6 carbon atoms and substituted or unsubstituted alkynyl having 2 to 6 carbon atoms, or
   R and R₁, when taken together, or R₂ and R₃, when taken together, constitute a substituted or unsubstituted 5- to 7-membered heterocyclic ring including the nitrogen atom to which they are attached, or R and R₂, when taken together, constitute a substituted or unsubstituted 5- to 7-membered heterocyclic ring including the guanidinium group to which they are attached, and
   R₄ represents a substituted or unsubstituted 6- to 10-membered aromatic or heteroaromatic ring, and
   X⁻ is an anion;
   or
ii) an uronium salt of formula (b) 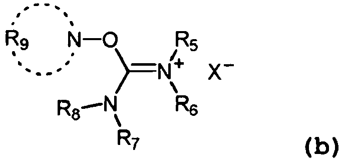
wherein
R₅, R₆, R₇, R₈ are independently selected from hydrogen, substituted or unsubstituted alkyl having 1 to 6 carbon atoms, substituted or unsubstituted alkenyl having 2 to 6 carbon atoms and substituted or unsubstituted alkynyl having 2 to 6 carbon atoms, or
R₅ and R₆, when taken together, or R₇ and R₈, when taken together, constitute a substituted or unsubstituted 5- to 7-membered heterocyclic ring including the nitrogen atom to which they are attached, or R₆ and R₇, when taken together, constitute a substituted or unsubstituted 5- to 7-membered heterocyclic ring including the uronium group to which they are attached,
R₉ represents a substituted or unsubstituted 5- to 7-membered heterocyclic ring including the nitrogen atom to which is attached, and
X⁻ is an anion, wherein said carboxylated labelling compound is a luminescent compound containing a carboxylic moiety

A further embodiment of the present invention concerns use of a mixture of said carboxylated labelling compound and a tertiary amine (Reagent A), and a guanidinium salt of formula (a) or a uronium salt of formula (b) (as defined above) (Reagent B) for labelling biomolecules bearing reactive amino or hydroxyl groups.

A still further embodiment of the present invention concerns a method for labelling biomolecules bearing reactive amino or hydroxyl groups with said carboxylated labelling compound, comprising i) providing a mixture of the carboxylated labelling compound and a tertiary amine (Reagent A), and one between a guanidinium salt of formula (a) or a uronium salt of formula (b) (as defined above) (Reagent B), ii) contacting the mixture and the guanidinium salt or the uronium salt obtaining an activated carboxylated labelling compound; iii) contacting the activated carboxylated labelling compound with the biomolecule obtaining a biomolecule labelled with the carboxylated labelling compound.

Both Reagent A and Reagent B are chemically stable for a very long period of time, when stored in the absence of moisture.

Reagent A can be a solution in a suitable solvent, such as a water miscible, polar aprotic solvent such as DMF, DMSO, and the like. However, it can be, in most cases evaporated to a solid.

Reagent B consists of a guanidinium or uronium coupling reagent (activator), such as N,N,N',N'-tetramethyl-O-(N-succinimidyl)uronium tetrafluoroborate (TSTU). These compounds are powerful activators well known in peptide synthesis, e.g., Knorr et al, Tet. Lett. 1990, 30, 1927; Bannwarth and Knorr, Tet. Lett. 1991, 32, 1157; Carpino et al. J. Org. Chem., 1994, 59, 695-698; US 6, 825, 347) either solid, or dissolved in a suitable solvent such as a water miscible, polar aprotic solvent such as DMF, DMSO, and the like.

At least one of the two reagents must be in solution form.

Upon contacting Reagent A and Reagent B, an activated form of the carboxylated labelling compound is rapidly formed, in general, quantitatively. This activated form can then be used to reliably and reproducibly label a biomolecule bearing reactive amino or hydroxyl groups. Reaction byproducts are easily removed by chromatography, dialysis, or ultrafiltration techniques.

### BRIEF DESCRIPTION OF THE FIGURES

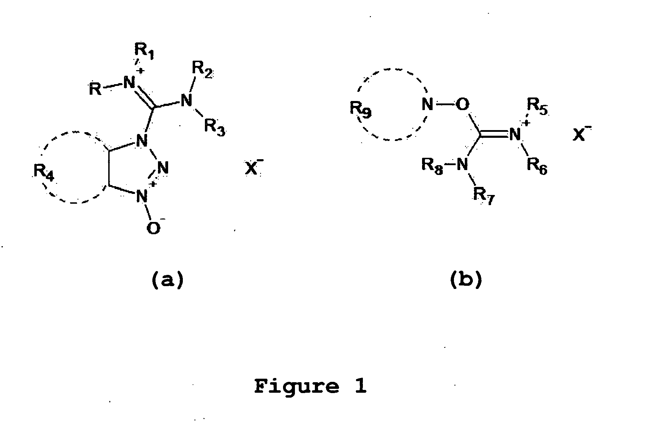

- Figure 1 illustrates the general formulae of the uronium (a) and guanidinium (b) salts used as activators according to the present invention.
- Figure 2 graphically illustrates the steps required for the use of the antibody labelling kits prepared according to the present invention, namely (a) Activated Dye Solution Preparation; (b) Antibody Solution Preparation; (c) Conjugation Procedure; (d) Purification and Isolation of the Conjugate.

### DETAILED DESCRIPTION OF THE INVENTION

The kits prepared according to the method of the present invention can be used in many biological and biotechnological procedures. For example, they can be used to label antibodies or other proteins. The labelled antibodies or proteins can be used as reagents in immunoassays or other diagnostic techniques such as flow cytometry, or in bioimaging, or as therapeutic reagents. The labelling kits can be successfully employed in proteomics techniques, such as differential gel electrophoresis.

Similarly, the kits of this invention can be used to label amino-modified nucleotides to be used in nucleic acid assays.

The kits prepared according to this invention overcome the difficulties of the presently used active ester, by providing the labelling compound, containing a carboxyl group, and the coupling reagent in separate packages, which can be stored for a prolonged period, without degradation. No weighing of costly, moisture sensitive material is required, nor aliquoting of rapidly decomposing solutions.

In more detail, the kit consists of a Reagent A and a Reagent B, individually packaged, comprising: a mixture of a carboxylated labelling compound and a tertiary amine (Reagent A); and a coupling reagent (Reagent B). The two reagents are carefully formulated and proportioned for a given amount of biomolecules to be labeled.

Reagent A consists of a mixture of a carboxylated compound and a tertiary amine, solid or in solution.

The carboxylated compound is a luminescent compound containing a carboxylic moiety, and said luminescent compound can belong to the class of coumarines, fluoresceines and rhodamines, polymethines including cyanines and merocyanines, oxazines, thiazines, phthalo- and naphthalocyanines, diazaindacenes, transition metal complexes and chelates, such as luminescent complexes of Ru (II), Os (II) and Ir (III) with aromatic diazines, luminescent complexes of Eu (III) and Tb, or

a carboxyl functionalized derivative of luminol and its analogues, e.g. hemisuccinamides of 5-amino-2,3-dihydrophthalazine-1,4-dione (or aminobutilisoluminol, ABEI), 6-amino-2,3-dihydrophthalazine-1,4-dione, 9-amino-2,3-dihydrobenzo[f]phthalazine-1,4-dione (or aminobutyl naphthol, ABEN), or other phthalazine derivatives, or carboxyl functionalized acridinium esters.

The tertiary amine of Reagent A can be triethylamine, N,N-diisopropylethylamine, pyridine, N,N-dimethylamino pyridine (DMAP), 4-piperidylpyridine (PPY), 2,4,6-trimethylpyridine (collidine), 4-methylmorpholine, 4-ethylmorpholine, 4-morpholinopyridine.

Reagent B is a coupling reagent comprising a guanidinium (a) or uronium (b) salt.

The guanidinium salt presents chemical formula (a): 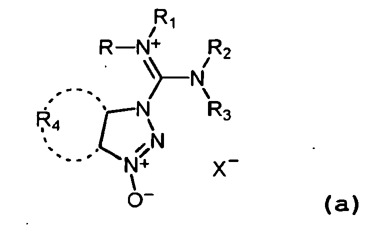 wherein
R, R₁, R₂, R₃ are independently selected from hydrogen, substituted or unsubstituted alkyl having 1 to 6 carbon atoms, substituted or unsubstituted alkenyl having 2 to 6 carbon atoms and substituted or unsubstituted alkynyl having 2 to 6 carbon atoms, or
R and R₁, when taken together, or R₂ and R₃, when taken together, constitute a substituted or unsubstituted 5- to 7-membered heterocyclic ring including the nitrogen atom to which they are attached, or R and R₂, when taken together, constitute a substituted or unsubstituted 5- to 7-membered heterocyclic ring including the guanidinium group to which they are attached,
R₄ represents a substituted or unsubstituted 6- to 10-membered aromatic and heteroaromatic ring, and
X⁻ is an anion.

The uronium salt presents chemical formula (b): 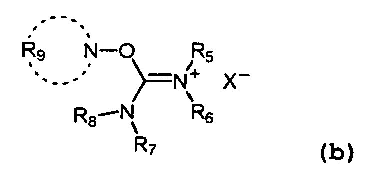 wherein
R₅, R₆, R₇, R₈ are independently selected from hydrogen, substituted or unsubstituted alkyl having 1 to 6 carbon atoms, substituted or unsubstituted alkenyl having 2 to 6 carbon atoms and substituted or unsubstituted alkynyl having 2 to 6 carbon atoms, or
R₅ and R₆, taken together, or R₇ and R₈, when taken together, constitute a substituted or unsubstituted 5-to 7-membered heterocyclic ring including the nitrogen atom to which they are attached, or R₆ and R₇, when taken together, constitute a substituted or unsubstituted 5- to 7-membered heterocyclic ring including the uronium group to which they are attached,
R₉ represents a substituted or unsubstituted 5- to 7-membered heterocyclic ring including the nitrogen atom to which is attached,
X⁻ is an anion.

Advantageously, the guanidinium or uronium salt is chosen among the following, commercially available compounds: O-(benzotriazol-1-yl)-N,N,N',N'-tetramethyl uronium hexafluorophosphate (HBTU), or 0-(benzotriazol-1-yl)-N,N,N',N'-tetramethyluronium tetrafluoroborate (TBTU), or 0-(7-azabenzotriazol-1-yl)-N,N,N',N'-tetramethyluronium hexafluorophosphate (HATU), or N,N,N',N'-tetramethyl-O-(N-succinimidyl)uronium tetrafluoroborate (TSTU), or N,N,N',N'-tetramethyl-0-(N-succinimidyl)uronium hexafluorophosphate (HSTU), (1-cyano-2-ethoxy-2-oxoethylidenaminooxy)dimethylamino-morpholin-carbenium exafluorophosphate (COMU). It is worth noting that the commonly used nomenclature for HATU, HBTU, TBTU compounds is misleading, since it attributes these compounds to the uronium structure, instead of the correct guanidinium structure of formula (a).

Reagent A and Reagent B are formulated either as solids or as solutions, and when they are formulated as solution the solvent is a polar aprotic, water miscible solvent selected from N,N-dimethylformamide, dimethylsulfoxide, N,N-dimethylacetamide, N-methylpyrrolidone, acetonitrile; at least one of the two reagents is formulated as a solution.

The amount of tertiary amine used in the formulation of reagent A is from 1:1 to 10:1 molar ratio with respect to the carboxylated labelling compound, while the amount of coupling reagent used in the formulation of reagent B is from 1:1 to 10:1 molar ratio with respect to the carboxylated labelling compound.

The kit components can be stored until needed for a prolonged period. Advantageously, the kit is proportioned for a specific amount of the biomolecule to be labelled, so that weighing or aliquoting is not required, and only the necessary amount of labelling compound is used.

Activation of the carboxylated labelling compound occurs upon contacting Reagent A and Reagent B. The activation is carried out at a temperature between 0 and 60 °C for up to 60 minutes.

An especially advantageous application of the labelling kit of the present invention is in the labelling of proteins for 2-D Gel Electrophoresis; see, e.g. "Proteomics" by Renders and Sickman, Eds., Methods in Molecular Biology, Vol. 564, 2009, Humana Press. For this application, in fact, tiny amounts of active esters of three monocationic, size matched dyes in DMF solution are used commercially. In this form the have very limited stability and poor reproducibility, since one dye (pentamethinindocyanine) is more hydrolytically and photochemical unstable than the other two dyes (trimethinindocyanine and trimethinoxacyanine).

These problems are avoided by using the kit of this invention, where the three dyes are stored in a stable carboxylic acid form, which is activated only when needed.

Further characteristics of the present invention will arise from the hereinafter description of some examples that are purely illustrative and not limiting.

### EXAMPLES

### Example 1

### Preparation of a kit for the labelling of antibodies with a fluorescent or luminogenic dye

The kit consists of two vials, one containing Reagent A, the other containing reagent B. The amount of reagents has been optimized for the labelling of 1 mg (6.7 nmol) of IgG (FR 150,000 daltons). Thus, the kit contains 15 equivalents of label (carboxylic acid) (100 nmol), 16.5 equivalents of 4-dimethylamino pyridine(DMAP) and 16.5 equivalents of *N,N,N',N'-*tetramethyl-*O*-(*N*-succinimidyl)uronium tetrafluoroborate (TSTU, 110 nmol) with respect to the IgG.

Reagent A (label + tertiary amine). Content: each 1.5 mL, conical bottom cryogenic vial with a o-ring seal cap contains 100 nmol of label (carboxylic acid) and 110 nmoles of DMAP.

Stock solutions are prepared by dissolving 2 µmoles of the label (carboxylic acid) in 1 mL of methanol or other volatile solvent and 1.07 mg (8.75 µmoles) of DMAP in 4 mL of methanol.

50 uL of each solution are added to a 1.5 mL conical bottom, cryogenic vial, and the solvent is evaporated using a rotary evaporator or an evaporative centrifuge.

The residue, which deposits as a film onto the bottom the vial, is further dried in vacuo.

The vial is then stored in the dark, under argon, at -20 °C, in a desiccator.

Reagent B (activator). Each 0.5 mL, conical bottom cryogenic vial with a o-ring seal cap contains 70 µL of a 3 mM solution of TSTU in dry DMF.

A stock solution is prepared by dissolving 3.31 mg (11 µmoles) of TSTU in 5 mL of dry DMF under argon. 70 µL aliquots are pipetted into 0.5 mL conical bottom, cryogenic vials, closed and stored under argon at - 20°C, in a desiccator.

The kit is assembled by placing the vials containing Reagent A and Reagent B together with a desiccant bag inside a barrier-film (aluminium/polyethylene) pouch, which is then heat sealed and stored at -20 °C.

### Example 2

### Labelling of an antibody with a fluorescent or luminogenic dye using the Kit of Example 1

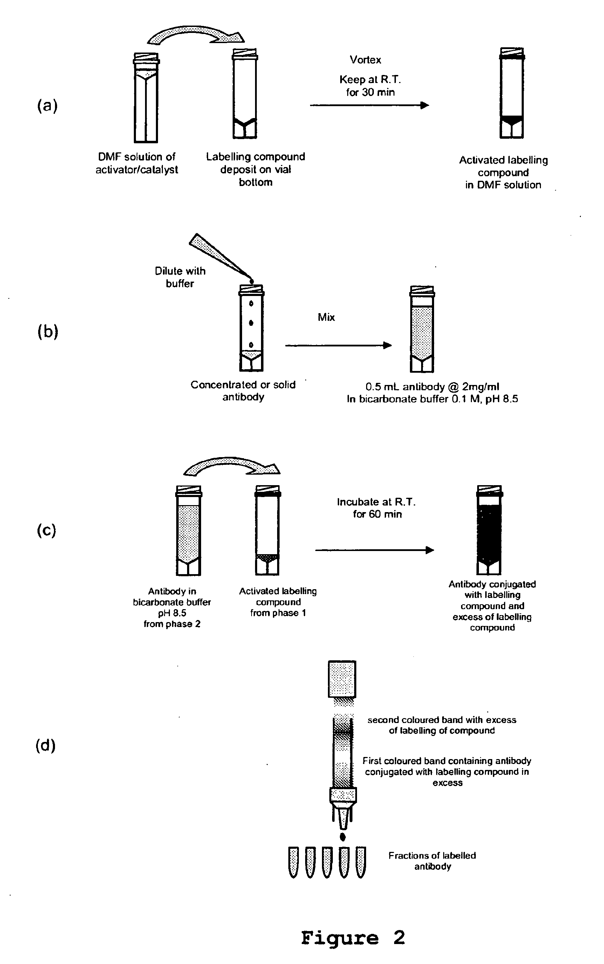

Step 1. Activated Dye Solution Preparation (Figure 2a). the kit is warmed to room temperature. 50 µL of Reagent B (activator solution) are added to the vial containing Reagent A. The vial is capped, and the dye dissolved by vortexing; the mixture is kept in the dark at room temperature for 30 min (Figure 2a).
Step 2. Antibody Solution Preparation (Figure 2b). Each kit is designed to label 1 mg of IgG (MW 150,000) at 2 mg/mL solution. The antibody must be dissolved in an amine-free buffer. For antibody buffer solution: to 450 uL of antibody diluted to 2.2 mg/mL with PBS buffer, 50 uL of 1M, pH 8.5 bicarbonate buffer are added. For solid antibodies: 1 mg of antibody is dissolved in 0.5 mL of 0.1 M, pH 8.5 bicarbonate buffer (Figure 2b).
Step 3. Conjugation Procedure (Figure 2c). The antibody solution in buffer (from step 2) is added to the vial containing the dye solution (from step 1). The vial is capped, gently mixed (without vortexing) and the solution is incubated at room temperature in the dark for 1 h, shaking every 15 min.
Step 4. Purification and Isolation of the Conjugate (Figure 2d). The conjugate product is separated from unreacted dye and reaction byproducts by gel permeation chromatography, e.g. on Sephadex™ G25. A chromatography column is equilibrated with PBS or the desired buffer, the reaction mixture from Step 3 is added, and eluted with the same buffer. The first colored band, containing the dye/antibody conjugate is collected. Alternatively one can use commercially available spin columns. Less efficient separation may be achieved by dialysis or ultrafiltration.

### Example 3

### Labelling of proteins for 2-D Fluorescence Gel Electrophoresis (2-D DIGE)

Labelling kits are specifically designed for proteins detection in 2-D Fluorescence Difference Gel Electrophoresis (2-D DIGE) applications. The minimal labelling approach requires a minimized concentration of the dye in order to label each protein with a single molecule of dye. Labelling occurs by forming a covalent bond between the ε-amino group of lysine in proteins and the activated dye.

For a typical minimal labelling 2D-DIGE analysis three dye labels are required, with approximate absorption wavelength at 490, 550 and 645 nm. The dyes must be:
- monocationic. An intrinsic positive charge on each dye replaces the one from lysine leaving the total protein charge unchanged.
- size-matched. All the dyes have similar molecular weight and shape to avoid affecting protein migration
- pH insensitive: no signal affection is observed over a wide range of pH.

In addition, high brightness of the dyes is required, for high sensitivity.

For example, dyes belonging to the class of cyanines can fulfil this requirement.

### Kit Preparation

Three separate kits are prepared, one for each dye. Each kit consists of two vials. One vial contains the dye/tertiary amine mixture, while the other contains the activator. For example, kits designed for 20 nmol of dye can be prepared as follows:
2-D DIGE Dye/tertiary amine Vial. Three DIGE dyes are used, a monocationic pentamethinindocyanine, a monocationic trimethinindocyanine and a monocationic trimethinoxacyanine, respectively. The following stock solutions are prepared: 1.08 mg of DMAP are dissolved in 10 mL of dry DMF; 4 micromoles of DIGE dye are introduced into an amber 5-mL volumetric flask, and 5 mL of the DMAP stock solution are added. The dye is dissolved, and the flask is capped under argon and stored at -20°C. 25 uL aliquots are pipetted into 0.5 mL conical bottom, cryogenic vials. The vials are stored in the dark with a desiccant under argon at -20 °C.

Activator Vial. Stock solution: 1.32 mg of TST are dissolved in 5 mL of anhydrous DM under argon in a 5 mL amber volumetric flask. in the dark with a desiccant under argon at -20 °C. 25 uL aliquots are pipetted into 0.5 mL conical bottom, cryogenic vials. The vials are stored in the dark with a desiccant under argon at -20 °C.

The kit is assembled by placing the two vials, together with a desiccant bag inside a barrier-film (aluminium/polyethylene) pouch, which is then heat sealed and stored at -20 °C.

Each 2-DIGE Minimal Labelling Kit contains 20 nmol of the 2-DIGE Dye dissolved in 25 µL of free-amine dry DMF, allowing perform up to 50 labelling reactions.

The working solution is obtained by mixing equal volumes of the two supplied solutions. A typical labelling reaction on 50 µg of protein requires 1 µL of the labelling working solution, obtained by mixing 0.5 µL of 2-D DIGE Dye and 0.5 µL of Activator. To minimize pipetting errors the labelling reaction may be scaled-up as required.

### Activation Procedure.

The kit is removed from the freezer and warmed up to room temperature for 5 minutes. The vials are inserted in a microcentrifuge and briefly spun to collect the solutions on the bottom of the vials. The labelling working solution is obtained as follows:
- equal volumes of 2-D DIGE Dye/tertiary amine and activator solution are pipetted into a new microcentrifuge tube;
- the tube is capped, mixed by vortexing and centrifuged for a few seconds, then kept the dark at room temperature for 30 minutes;
- the labelling working solution will have a concentration of 400 pmol/µL in the active dye.

The working solution is typically prepared immediately before use. If necessary, the working solution can be stored at -20 °C for up to one week.

### Labelling Protocol

The recommended protein concentration in the cell lysate is in the range 5-10 mg/mL, so that 50 µg of protein are portioned in 10 and 5 µL respectively, but also samples containing 1 mg/mL of proteins may be labeled as well.

pH of the protein solution has to be 8.5 to guarantee the maximum efficiency.

To label 50 µg of protein 400 pmol of the active dye are recommended. Proceed as follow:
- place the cell lysate solution containing 50 µg of protein in a microcentrifuge tube;
- add 1 µL of the labelling working solution containing 400 pmol of the active dye to the same tube;
- mix by pipetting up and down and centrifuge briefly;
- incubate on ice for about 30 minutes in the darkness;
- quench the labelling reaction by adding 1 µL of 10 mM lysine solution: mix by vortexing and centrifuge for few seconds, then keep in the dark on ice for 10 minutes.

Labelled protein solution is now ready to be processed, otherwise store at -70°C in the darkness.

### Example 4

### Labelling of amino-modified oligonucleotides

A labelling kit was specifically developed to offer ideal conditions for the labelling of amino allyl modified DNA and RNA strands. The amount of label for each kit is adjusted to give an optimal 20-50 label molecules per 1000 nucleotides.

The kit consists of two vials, one containing Reagent A, the other containing reagent B. The amount of reagents has been optimized for the labelling of 5-20 mg (6.7 nmol) of aminoallyl arNA; aRNA = antisenseRNA. Thus, the kit contains 40 nmol of carboxylic acid label, 60 nmol of 4-dimethylaminopyridine(DMAP) and 60 nmol of *N,*N*,N',N'*-tetramethyl-*O-*(N-succinimidyl) uronium tetrafluoborate (TSTU) in dry DMF.

Reagent A (label + tertiary amine). Content: each 0.5 mL, DNase e RNase free, conical bottom, cryogenic vial with a o-ring seal cap contains 40 nmol of label (carboxylic acid) and 60 nmoles of DMAP. A stock solution is prepared by dissolving 7.2 µmoles of carboxylic acid label and 1.31 mg of DMAP in 5 mL of methanol in a 5 mL amber vial. 27.8 µL aliquots of this solution are pipetted into a 0.5 mL, conical bottom, cryogenic vial, and the solvent is evaporated using a rotary evaporator or an evaporative centrifuge. The residue, which deposits as a film onto the bottom the vial, is further dried in vacuo. The vial is then stored in the dark, under argon, at -20 °C, in a dessicator.

Reagent B (activator). Content: each 0.5 mL, DNase e RNase free, conical bottom, cryogenic vial with a o-ring seal cap contains 20 µL a TSTU solution. A stock solution is prepared by dissolving 8.20 mg of TSTU in 5 mL of dry DMF, under argon, in a 5 ml glass vial. 20.0 µL aliquots of this solution are pipetted into a 0.5 mL, conical bottom, cryogenic vial. The vial is then stored in the dark, under argon, at -20 °C, in a dessicator.

The kit is assembled by placing the vials containing Reagent A and Reagent B together with a dessicant bag inside a barrier-film (aluminium/polyethylene) pouch, which is then heat sealed and stored at -20 °C.

Labelling protocol - The following protocol was developed for labelling amino allyl modified nucleotides using the labelling kit.
Step 1 - Activated Label Solution Preparation. Let the kit warm to room temperature. Add 11 µL of Reagent B (activator) solution to the Reagent A (label + tertiary amine) vial. Cap the vial, dissolve the dye by vortexing and keep in the dark at room temperature for 30 minutes.
Step 2 - aRNA Solution Preparation. Each kit is designed to label room temperature. Each kit is designed to label 5-20 µg of amino allyl modified aRNA. Dry the desired amount of aRNA sample, using for example a vacuum centrifuge. Make sure not to overdry. Add 9 µL of coupling buffer (0.1 M sodium bicarbonate solution pH 8.7).
Step 3 - Conjugation Procedure. Add the entire activated CHROMIS dye (11 µL from step 1) to the aRNA tube to bring the total volume to 20 µL. Pipette several times to mix well and incubate in the dark at room temperature for at least 30-60 minutes. To quench the reaction add 4.5 µL of a 4 M hydroxylamine solution and incubate in the dark at room temperature for 15 minutes. Proceed to purification of labelled aRNA probes by HPLC or using suitable purification cartridges and protocols.

Of course, without prejudice to the underlying principle of the invention, the details of construction and the embodiments may vary, even to a great extent, with respect to what has been described and illustrated purely by way of example, without departing from the scope of the present invention as defined by the annexed claims.

## Claims

1. A kit for labelling biomolecules bearing reactive amino or hydroxyl groups comprising a first component (Reagent A) and a second component (Reagent B) in separate packages, wherein said first component comprises a mixture of a carboxylated labelling compound and a tertiary amine, and wherein said second component comprises:
i) a guanidinium salt of formula (a) 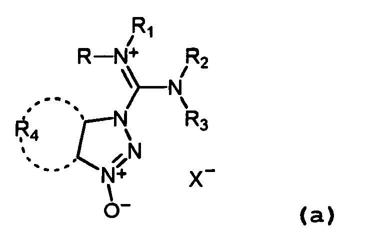 wherein
R, R₁, R₂, R₃ are independently selected from hydrogen, substituted or unsubstituted alkyl having 1 to 6 carbon atoms, substituted or unsubstituted alkenyl having 2 to 6 carbon atoms and substituted or unsubstituted alkynyl having 2 to 6 carbon atoms, or
R and R₁, when taken together, or R₂ and R₃, when taken together, constitute a substituted or unsubstituted 5- to 7-membered heterocyclic ring including the nitrogen atom to which they are attached, or
R and R₂, when taken together, constitute a substituted or unsubstituted 5- to 7-membered heterocyclic ring including the guanidinium group to which they are attached, and
R₄ represents a substituted or unsubstituted 6- to 10-membered aromatic and heteroaromatic ring, and
X⁻ is an anion;
or
ii) an uronium salt of formula (b) 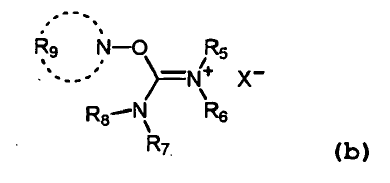 wherein
R₅, R₆, R₇, R₈ are independently selected from hydrogen, substituted or unsubstituted alkyl having 1 to 6 carbon atoms, substituted or unsubstituted alkenyl having 2 to 6 carbon atoms and substituted or unsubstituted alkynyl having 2 to 6 carbon atoms, or
R₅ and R₆, taken together, or R₇ and R₈, when taken together, constitute a substituted or unsubstituted 5-to 7-membered heterocyclic ring including the nitrogen atom to which they are attached, or R₆ and R₇, when taken together, constitute a substituted or unsubstituted 5- to 7-membered heterocyclic ring including the uronium group to which they are attached, and
R₉ represents a substituted or unsubstituted 5- to 7-membered heterocyclic ring including the nitrogen atom to which is attached, and
X⁻ is an anion,
wherein said carboxylated labelling compound is a luminescent compound containing a carboxylic moiety.

2. The kit according to claim 1, wherein said luminescent compound is selected from Coumarines *containing a carboxylic moiety*, fluoresceines *containing a carboxylic moiety* and rhodamines *containing a carboxylic moiety*, polymethines *containing a carboxylic moiety* including cyanines, *merocyanines*, oxazines, thiazines, phthalo- and naphthalocyanines, diazaindacenes, amino substituted 2,3-dihydrophthalazine-1,4-diones, amino substituted 2,3-dihydrobenzo[*f*]phthalazine-1,4-diones, acridinium esters *containing a carboxylic moiety,* luminescent complexes of Ru (II), Os (II) and Ir (III) with aromatic diazines *containing* a *carboxylic moiety,* luminescent complexes of Eu (III) and Tb (III) *containing a carboxylic moiety, a carboxyl functionalized derivative of luminol and its analogues.*

3. The kit according to any one of the preceding claims, wherein said tertiary amine is selected from triethylamine, N,N-diisopropylethylamine, pyridine, N,N-dimethylamino pyridine, 2,4,6-trimethylpyridine (collidine), 4-methylmorpholine, 4-ethylmorpholine, 4-morpholinopyridine.

4. The kit according to any one of the preceding claims, wherein said first component and said second component are formulated either as solids or solutions.

5. The kit according to claim 4, wherein said first component and said second component are formulated as solutions, the solvent being selected from N,N-dimethylformamide, dimethylsulfoxide, sulfolane, N,N-dimethylacetamide, N-methylpyrrolidone, acetonitrile.

6. The kit according to any one of the preceding claims, wherein the amount of said second component is in 1:1 to 10:1 molar ratio with respect to said carboxylated labelling compound.

7. The kit according to any one of the preceding claims, wherein said guanidinium salt of formula (a) is selected from *O*-(benzotriazol-1-yl)-N,N,N',N'-tetramethyl uronium hexafluorophosphate (HBTU), O-(benzotriazol-1-yl)-N,N,N',N',N'-tetramethyluronium tetrafluoroborate (TBTU), O-(7-azabenzotriazol-1-yl)-N,N,N',N'-tetramethyluronium hexafluorophosphate (HATU).

8. The kit according to any one of claims 1 to 7, wherein said uronium salt of formula (b) is selected from N,N,N',N'-tetramethyl-O-(N-succinimidyl)uronium tetrafluoroborate (TSTU), or N,N,N',N'-tetramethyl-0-(N-succinimidyl)uronium hexafluorophosphate (HSTU), (1-cyano-2-ethoxy-2-oxoethylidenaminooxy)dimethylamino-morpholin-carbenium exafluorophosphate (COMU).

9. Use of a carboxylated labelling compound, a tertiary amine and one compound between
i) a guanidinium salt of formula (a) 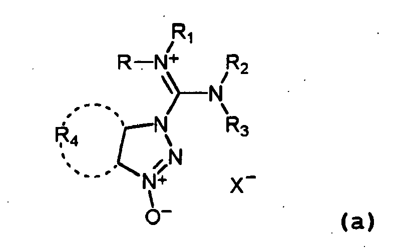 wherein
R, R₁, R₂, R₃ are independently selected from hydrogen, substituted or unsubstituted alkyl having 1 to 6 carbon atoms, substituted or unsubstituted alkenyl having 2 to 6 carbon atoms and substituted or unsubstituted alkynyl having 2 to 6 carbon atoms, or
R and R₁, when taken together, or R₂ and R₃, when taken together, constitute a substituted or unsubstituted 5- to 7-membered heterocyclic ring including the nitrogen atom to which they are attached, or
R and R₂, when taken together, constitute a substituted or unsubstituted 5- to 7-membered heterocyclic ring including the guanidinium group to which they are attached, and
R₄ represents a substituted or unsubstituted 6- to 10-membered aromatic and heteroaromatic ring, and
X⁻ is an anion;
or
ii) an uronium salt of formula (b) 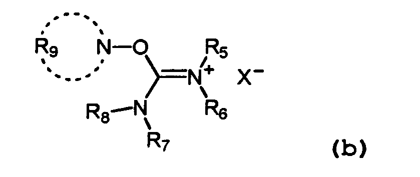 wherein
R₅, R₆, R₇, R₈ are independently selected from hydrogen, substituted or unsubstituted alkyl having 1 to 6 carbon atoms, substituted or unsubstituted alkenyl having 2 to 6 carbon atoms and substituted or unsubstituted alkynyl having 2 to 6 carbon atoms, or
R₅ and R₆, taken together, or R₇ and R₈, when taken together, constitute a substituted or unsubstituted 5-to 7-membered heterocyclic ring including the nitrogen atom to which they are attached, or R₆ and R₇, when taken together, constitute a substituted or unsubstituted 5- to 7-membered heterocyclic ring including the uronium group to which they are attached, and
R₉ represents a substituted or unsubstituted 5- to 7-membered heterocyclic ring including the nitrogen atom to which is attached, and
X⁻ is an anion,
for labelling biomolecules bearing reactive amino or hydroxyl groups, wherein said carboxylated labelling compound is a luminescent compound containing a carboxylic moiety.

10. Use according to claim 9, wherein said luminescent compound is selected from coumarines *containing a carboxylic moiety,* fluoresceines *containing a carboxylic moiety* and rhodamines *containing a carboxylic moiety,* polymethines *containing a carboxylic moiety* including cyanines, merocyanines, oxazines, thiazines, phthalo- and naphthalocyanines, diazaindacenes, amino substituted 2,3-dihydrophthalazine-1,4-diones, amino substituted 2,3-dihydrobenzo[f]phthalazine-1,4-diones, acridinium esters *containing a carboxylic moiety,* luminescent complexes of Ru (II), Os (II) and Ir (III) with aromatic diazines *containing a carboxylic moiety,* luminescent complexes of Eu (III) and Tb (III) *containing a carboxylic moiety, a carboxyl functionalized derivative of luminol and its analogues.*

11. Use according to any one of claims 9 to 10, wherein said tertiary amine is selected from triethylamine, N,N-diisopropylethylamine, pyridine, N,N-dimethylamino pyridine, 2,4,6-trimethylpyridine (collidine), 4-methylmorpholine, 4-ethylmorpholine, 4-morpholinopyridine.

12. Use according to any one of claims 9 to 11, wherein said guanidinium salt of formula (a) is selected from O-(benzotriazol-1-yl)-N,N,N',N'-tetramethyl uronium hexafluorophosphate (HBTU); O-(benzotriazol-1-yl)-N,N,N',N'-tetramethyluronium tetrafluoroborate (TBTU), O-(7-azabenzotriazol-1-yl)-N,N,N',N'-tetramethyluronium hexafluorophosphate (HATU).

13. Use according to any one of claims 9 to 11, wherein said uronium salt of formula (b) is selected from N,N,N',N'-tetramethyl-O-(N-succinimidyl)uronium tetrafluoroborate (TSTU), or N,N,N',N'-tetramethyl-O-(N-succinimidyl)uronium hexafluorophosphate (HSTU), (1-cyano-2-ethoxy-2-oxoethylidenaminooxy)dimethylamino-morpholin-carbenium exafluorophosphate (COMU).

14. A method for labelling biomolecules bearing reactive amino or hydroxyl groups with a carboxylated labelling compound, comprising
i) providing in separate packages a mixture of the carboxylated labelling compound and a tertiary amine, and one between:
- a guanidinium salt of formula (a) 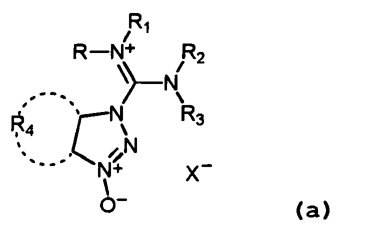 wherein
R, R₁, R₂, R₃ are independently selected from hydrogen, substituted or unsubstituted alkyl having 1 to 6 carbon atoms, substituted or unsubstituted alkenyl having 2 to 6 carbon atoms and substituted or unsubstituted alkynyl having 2 to 6 carbon atoms, or
R and R₁, when taken together, or R₂ and R₃, when taken together, constitute a substituted or unsubstituted 5- to 7-membered heterocyclic ring including the nitrogen atom to which they are attached,
or
R and R₂, when taken together, constitute a substituted or unsubstituted 5- to 7-membered heterocyclic ring including the guanidinium group to which they are attached, and
R₄ represents a substituted or unsubstituted 6- to 10-membered aromatic and heteroaromatic ring, and
X⁻ is an anion;
or
- a uronium salt of formula (b) 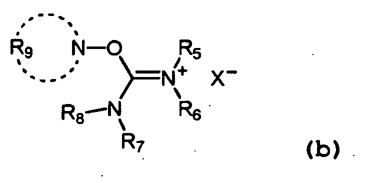 wherein
R₅, R₆, R₇, R₈ are independently selected from hydrogen, substituted or unsubstituted alkyl having 1 to 6 carbon atoms, substituted or unsubstituted alkenyl having 2 to 6 carbon atoms and substituted or unsubstituted alkynyl having 2 to 6 carbon atoms, or
R₅ and R₆, taken together, or R₇ and R₈, when taken together, constitute a substituted or unsubstituted 5-to 7-membered heterocyclic ring including the nitrogen atom to which they are attached, or R₆ and R₇, when taken together, constitute a substituted or unsubstituted 5- to 7-membered heterocyclic ring including the uronium group to which they are attached, and
R₉ represents a substituted or unsubstituted 5- to 7-membered heterocyclic ring including the nitrogen atom to which is attached, and
X⁻ is an anion;
ii) contacting the mixture and the guanidinium salt or the uronium salt obtaining an activated carboxylated labelling compound;
iii) contacting the activated carboxylated labelling compound with the biomolecule obtaining a biomolecule labelled with the carboxylated labelling compound
wherein the carboxylated labelling compound is a luminescent compound containing a carboxylic moiety.

15. The method according to claim 14, wherein said operation ii) is performed at a temperature between 0 and 60 °C for a period of time up to 60 minutes.

16. The method according to claim 14 or claim 15, wherein said operation iii) is performed at a temperature between 4 and 50 °C for a period of time up to 24 hours.

## Patentansprüche

1. Kit zum Markieren von Biomolekülen, die reaktive Amino- oder Hydroxylgruppen tragen, umfassend eine erste Komponente (Reagens A) und eine zweite Komponente (Reagens B) in getrennten Packungen, wobei die erste Komponente eine Mischung aus einer carboxylierten Markierungsverbindung und einem tertiären Amin umfasst, und wobei die zweite Komponente umfasst:
i) ein Guanidiniumsalz der Formel (a) 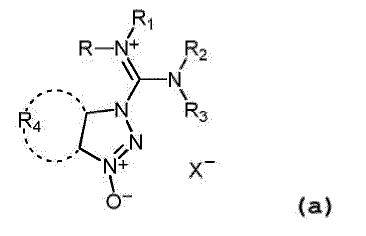 wobei
R, R₁, R₂, R₃ sind unabhängig ausgewählt aus Wasserstoff, substituiertem oder unsubstituiertem Alkyl mit 1 bis 6 Kohlenstoffatomen, substituiertem oder unsubstituiertem Alkenyl mit 2 bis 6 Kohlenstoffatomen und substituiertem oder unsubstituiertem Alkinyl mit 2 bis 6 Kohlenstoffatomen, oder
R und R₁, wenn sie zusammengefasst sind, oder R₂ und R₃, wenn sie zusammengefasst sind, bilden einen substituierten oder unsubstituierten 5- bis 7-gliedrigen heterocyclischen Ring, der das Stickstoffatom einschließt, an das sie gebunden sind, oder
R und R₂, wenn sie zusammengefasst sind, bilden einen substituierten oder unsubstituierten 5- bis 7-gliedrigen heterocyclischen Ring, der die Guanidiniumgruppe einschließt, an die sie gebunden sind, und
R₄ bedeutet einen substituierten oder unsubstituierten 6- bis 10-gliedrigen aromatischen und heteroaromatischen Ring, und
X⁻ ist ein Anion;
oder
ii) ein Uroniumsalz der Formel (b) 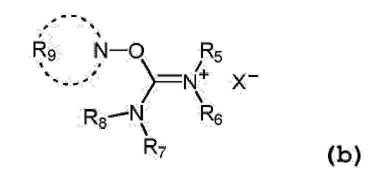 wobei
R₅, R₆, R₇, R₈ sind unabhängig ausgewählt aus Wasserstoff, substituiertem oder unsubstituiertem Alkyl mit 1 bis 6 Kohlenstoffatomen, substituiertem oder unsubstituiertem Alkenyl mit 2 bis 6 Kohlenstoffatomen und substituiertem oder unsubstituiertem Alkinyl mit 2 bis 6 Kohlenstoffatomen, oder
R₅ und R₆, zusammengefasst, oder R₇ und R₈, wenn sie zusammengefasst sind, bilden einen substituierten oder unsubstituierten 5- bis 7-gliedrigen heterocyclischen Ring, der das Stickstoffatom einschließt, an das sie gebunden sind, oder R₆ und R₇, wenn sie zusammengefasst sind, bilden einen substituierten oder unsubstituierten 5- bis 7-gliedrigen heterocyclischen Ring, der die Uroniumgruppe einschließt, an die sie gebunden sind, und
R₉ bedeutet einen substituierten oder unsubstituierten 5- bis 7-gliedrigen heterocyclischen Ring, der das Stickstoffatom einschließt, an das es gebunden ist, und
X⁻ ist ein Anion,
wobei die carboxylierte Markierungsverbindung eine lumineszierende Verbindung ist, die eine Carboxyleinheit enthält.

2. Kit nach Anspruch 1, wobei die lumineszierende Verbindung ausgewählt ist aus Cumarinen, die eine Carboxyleinheit enthalten, Fluoresceinen, die eine Carboxyleinheit enthalten, und Rhodaminen, die eine Carboxyleinheit enthalten, Polymethinen, die eine Carboxyleinheit enthalten, einschließlich Cyaninen, Merocyaninen, Oxazinen, Thiazinen, Phthalo- und Naphthalocyaninen, Diazaindacenen, Amino-substituierten 2,3-Dihydrophthalazin-1,4-dionen, Amino-substituierten 2,3-Dihydrobenzo[f]phthalazin-1,4-dionen, Acridiniumestern, die eine Carboxyleinheit enthalten, lumineszierenden Komplexen von Ru (II), Os (II) und Ir (III) mit aromatischen Diazinen, die eine Carboxyleinheit enthalten, lumineszierenden Komplexen von Eu (III) und Tb (III), die eine Carboxyleinheit enthalten, einem Carboxyl-funktionalisierten Derivat von Luminol und seinen Analoga.

3. Kit nach einem der vorangehenden Ansprüche, wobei das tertiäre Amin ausgewählt ist aus Triethylamin, N,N-Diisopropylethylamin, Pyridin, N,N-Dimethylaminopyridin, 2,4,6-Trimethylpyridin (Collidin), 4-Methylmorpholin, 4-Ethylmorpholin, 4-Morpholinopyridin.

4. Kit nach einem der vorangehenden Ansprüche, wobei die erste Komponente und die zweite Komponente entweder als Feststoffe oder Lösungen formuliert sind.

5. Kit nach Anspruch 4, wobei die erste Komponente und die zweite Komponente als Lösungen formuliert sind, wobei das Lösungsmittel ausgewählt ist aus N,N-Dimethylformamid, Dimethylsulfoxid, Sulfolan, N,N-Dimethylacetamid, N-Methylpyrrolidon, Acetonitril.

6. Kit nach einem der vorangehenden Ansprüche, wobei die Menge der zweiten Komponente in einem Molverhältnis von 1:1 bis 10:1, bezogen auf die carboxylierte Markierungsverbindung, vorliegt.

7. Kit nach einem der vorangehenden Ansprüche, wobei das Guanidiniumsalz der Formel (a) ausgewählt ist aus O-(Benzotriazol-1-yl)-N,N,N',N'-tetramethyluronium-hexafluorphosphat (HBTU), O-(Benzotriazol-1-yl)-N,N,N',N'-tetramethyluronium-tetrafluorborat (TBTU), O-(7-Azabenzotriazol-1-yl)-N,N,N',N'-tetramethyluronium-hexafluorphosphat (HATU).

8. Kit nach einem der Ansprüche 1 bis 7, wobei das Uroniumsalz der Formel (b) ausgewählt ist aus N,N,N',N'-Tetramethyl-O-(N-succinimidyl)uronium-tetrafluorborat (TSTU) oder N,N,N',N'-Tetramethyl-O-(N-succinimidyl)uronium-hexafluorphosphat (HSTU), (1-Cyano-2-ethoxy-2-oxoethylidenaminooxy)dimethylaminomorpholin-carbenium-hexafluorphosphat (COMU).

9. Verwendung einer carboxylierten Markierungsverbindung, eines tertiären Amins und einer Verbindung ausgewählt aus
i) einem Guanidiniumsalz der Formel (a) 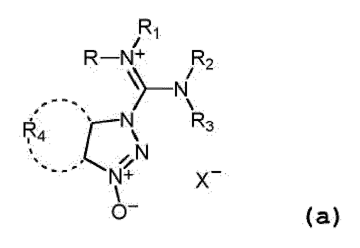 wobei
R, R₁, R₂, R₃ sind unabhängig ausgewählt aus Wasserstoff, substituiertem oder unsubstituiertem Alkyl mit 1 bis 6 Kohlenstoffatomen, substituiertem oder unsubstituiertem Alkenyl mit 2 bis 6 Kohlenstoffatomen und substituiertem oder unsubstituiertem Alkinyl mit 2 bis 6 Kohlenstoffatomen, oder
R und R₁, wenn sie zusammengefasst sind, oder R₂ und R₃, wenn sie zusammengefasst sind, bilden einen substituierten oder unsubstituierten 5- bis 7-gliedrigen heterocyclischen Ring, der das Stickstoffatom einschließt, an das sie gebunden sind, oder
R und R₂, wenn sie zusammengefasst sind, bilden einen substituierten oder unsubstituierten 5- bis 7-gliedrigen heterocyclischen Ring, der die Guanidiniumgruppe einschließt, an die sie gebunden sind, und
R₄ bedeutet einen substituierten oder unsubstituierten 6- bis 10-gliedrigen aromatischen und heteroaromatischen Ring, und
X⁻ ist ein Anion;
oder
ii) einem Uroniumsalz der Formel (b) 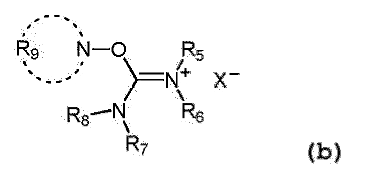 wobei
R₅, R₆, R₇, R₈ sind unabhängig ausgewählt aus Wasserstoff, substituiertem oder unsubstituiertem Alkyl mit 1 bis 6 Kohlenstoffatomen, substituiertem oder unsubstituiertem Alkenyl mit 2 bis 6 Kohlenstoffatomen und substituiertem oder unsubstituiertem Alkinyl mit 2 bis 6 Kohlenstoffatomen, oder
R₅ und R₆, zusammengefasst, oder R₇ und R₈, wenn sie zusammengefasst sind, bilden einen substituierten oder unsubstituierten 5- bis 7-gliedrigen heterocyclischen Ring, der das Stickstoffatom einschließt, an das sie gebunden sind, oder R₆ und R₇, wenn sie zusammengefasst sind, bilden einen substituierten oder unsubstituierten 5- bis 7-gliedrigen heterocyclischen Ring, der die Uroniumgruppe einschließt, an die sie gebunden sind, und
R₉ bedeutet einen substituierten oder unsubstituierten 5- bis 7-gliedrigen heterocyclischen Ring, der das Stickstoffatom einschließt, an das es gebunden ist, und
X⁻ ist ein Anion,
zum Markieren von Biomolekülen, die reaktive Amino- oder Hydroxylgruppen tragen, wobei die carboxylierte Markierungsverbindung eine lumineszierende Verbindung ist, die eine Carboxyleinheit enthält.

10. Verwendung nach Anspruch 9, wobei die lumineszierende Verbindung ausgewählt ist aus Cumarinen, die eine Carboxyleinheit enthalten, Fluoresceinen, die eine Carboxyleinheit enthalten, und Rhodaminen, die eine Carboxyleinheit enthalten, Polymethinen, die eine Carboxyleinheit enthalten, einschließlich Cyaninen, Merocyaninen, Oxazinen, Thiazinen, Phthalo- und Naphthalocyaninen, Diazaindacenen, Amino-substituierten 2,3-Dihydrophthalazin-1,4-dionen, Amino-substituierten 2,3-Dihydrobenzo[f]phthalazin-1,4-dionen, Acridiniumestern, die eine Carboxyleinheit enthalten, lumineszierenden Komplexen von Ru (II), Os (II) und Ir (III) mit aromatischen Diazinen, die eine Carboxyleinheit enthalten, lumineszierenden Komplexen von Eu (III) und Tb (III), die eine Carboxyleinheit enthalten, einem Carboxyl-funktionalisierten Derivat von Luminol und seinen Analoga.

11. Verwendung nach einem der Ansprüche 9 bis 10, wobei das tertiäre Amin ausgewählt ist aus Triethylamin, N,N-Diisopropylethylamin, Pyridin, N,N-Dimethylaminopyridin, 2,4,6-Trimethylpyridin (Collidin), 4-Methylmorpholin, 4-Ethylmorpholin, 4-Morpholinopyridin.

12. Verwendung nach einem der Ansprüche 9 bis 11, wobei das Guanidiniumsalz der Formel (a) ausgewählt ist aus O-(Benzotriazol-1-yl)-N,N,N',N'-tetramethyluronium-hexafluorphosphat (HBTU), O-(Benzotriazol-1-yl)-N,N,N',N'-tetramethyluronium-tetrafluorborat (TBTU), O-(7-Azabenzotriazol-1-yl)-N,N,N',N'-tetramethyluronium-hexafluorphosphat (HATU).

13. Verwendung nach einem der Ansprüche 9 bis 11, wobei das Uroniumsalz der Formel (b) ausgewählt ist aus N,N,N',N'-Tetramethyl-O-(N-succinimidyl)uronium-tetrafluorborat (TSTU) oder N,N,N',N'-Tetramethyl-O-(N-succinimidyl)uronium-hexafluorphosphat (HSTU), (1-Cyano-2-ethoxy-2-oxoethylidenaminooxy)dimethylaminomorpholin-carbenium-hexafluorphosphat (COMU).

14. Verfahren zum Markieren von Biomolekülen, die reaktive Amino- oder Hydroxylgruppen tragen, mit einer carboxylierten Markierungsverbindung, umfassend:
i) das Bereitstellen, in getrennten Packungen, von einer Mischung aus der carboxylierten Markierungsverbindung und einem tertiären Amin, und einem von:
- einem Guanidiniumsalz der Formel (a) 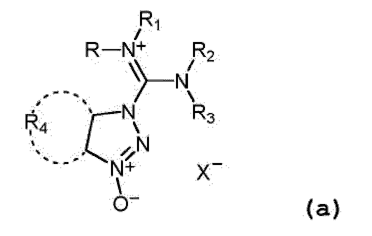 wobei
R, R₁, R₂, R₃ sind unabhängig ausgewählt aus Wasserstoff, substituiertem oder unsubstituiertem Alkyl mit 1 bis 6 Kohlenstoffatomen, substituiertem oder unsubstituiertem Alkenyl mit 2 bis 6 Kohlenstoffatomen und substituiertem oder unsubstituiertem Alkinyl mit 2 bis 6 Kohlenstoffatomen, oder
R und R₁, wenn sie zusammengefasst sind, oder R₂ und R₃, wenn sie zusammengefasst sind, bilden einen substituierten oder unsubstituierten 5- bis 7-gliedrigen heterocyclischen Ring, der das Stickstoffatom einschließt, an das sie gebunden sind, oder
R und R₂, wenn sie zusammengefasst sind, bilden einen substituierten oder unsubstituierten 5- bis 7-gliedrigen heterocyclischen Ring, der die Guanidiniumgruppe einschließt, an die sie gebunden sind, und
R₄ bedeutet einen substituierten oder unsubstituierten 6- bis 10-gliedrigen aromatischen und heteroaromatischen Ring, und
X⁻ ist ein Anion;
oder
- einem Uroniumsalz der Formel (b) 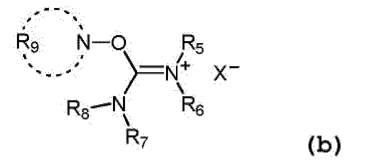 wobei
R₅, R₆, R₇, R₈ sind unabhängig ausgewählt aus Wasserstoff, substituiertem oder unsubstituiertem Alkyl mit 1 bis 6 Kohlenstoffatomen, substituiertem oder unsubstituiertem Alkenyl mit 2 bis 6 Kohlenstoffatomen und substituiertem oder unsubstituiertem Alkinyl mit 2 bis 6 Kohlenstoffatomen, oder
R₅ und R₆, zusammengefasst, oder R₇ und R₈, wenn sie zusammengefasst sind, bilden einen substituierten oder unsubstituierten 5- bis 7-gliedrigen heterocyclischen Ring, der das Stickstoffatom einschließt, an das sie gebunden sind, oder R₆ und R₇, wenn sie zusammengefasst sind, bilden einen substituierten oder unsubstituierten 5- bis 7-gliedrigen heterocyclischen Ring, der die Uroniumgruppe einschließt, an die sie gebunden sind, und
R₉ bedeutet einen substituierten oder unsubstituierten 5- bis 7-gliedrigen heterocyclischen Ring, der das Stickstoffatom einschließt, an das es gebunden ist, und
X⁻ ist ein Anion,
ii) das Inkontaktbringen der Mischung und des Guanidiniumsalzes oder des Uroniumsalzes, wobei eine aktivierte carboxylierte Markierungsverbindung erhalten wird;
iii) das Inkontaktbringen der aktivierten carboxylierten Markierungsverbindung mit dem Biomolekül, wobei ein mit der carboxylierten Markierungsverbindung markiertes Biomolekül erhalten wird,
wobei die carboxylierte Markierungsverbindung eine lumineszierende Verbindung ist, die eine Carboxyleinheit enthält.

15. Verfahren nach Anspruch 14, wobei der Arbeitsgang ii) bei einer Temperatur zwischen 0 und 60 °C während eines Zeitraums von bis zu 60 Minuten durchgeführt wird.

16. Verfahren nach Anspruch 14 oder Anspruch 15, wobei der Arbeitsgang iii) bei einer Temperatur zwischen 4 und 50 °C während eines Zeitraums von bis zu 24 Stunden durchgeführt wird.

## Revendications

1. Trousse pour marquer des biomolécules portant des groupes réactifs amino ou hydroxyle, comprenant un premier composant (réactif A) et un deuxième composant (réactif B) dans des conditionnements séparés, dans laquelle ledit premier composant comprend un mélange d'un composé de marquage carboxylé et d'une amine tertiaire, et dans laquelle ledit deuxième composant comprend :
i) un sel de guanidinium de formule (a) : 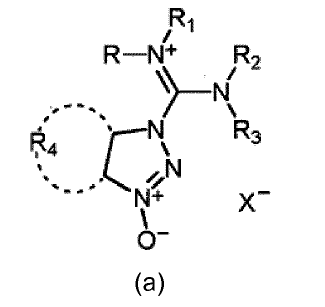 dans laquelle
R, R₁, R₂, R₃ sont indépendamment choisis parmi l'hydrogène et les radicaux alkyle substitués ou non substitués ayant de 1 à 6 atomes de carbone, alcényle substitués ou non substitués ayant de 2 à 6 atomes de carbone et alcynyle substitués ou non substitués ayant de 2 à 6 atomes de carbone, ou bien
R et R₁ pris ensemble, ou R₂ et R₃ pris ensemble, forment un hétérocycle substitué ou non substitué à 5 à 7 chaînons contenant l'atome d'azote auquel ils sont rattachés, ou bien
R et R₂ pris ensemble, forment un hétérocycle substitué ou non substitué à 5 à 7 chaînons contenant le groupe guanidinium auquel ils sont rattachés, et
R₄ représente un cycle aromatique ou hétéroaromatique substitué ou non substitué, à 6 à 10 chaînons, et
X⁻ est un anion ;
ou
ii) un sel d'uronium de formule (b) : 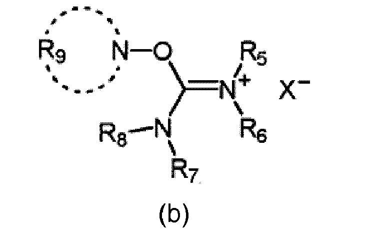 dans laquelle
R₅, R₆, R₇, R₈ sont indépendamment choisis parmi l'hydrogène et les radicaux alkyle substitués ou non substitués ayant de 1 à 6 atomes de carbone, alcényle substitués ou non substitués ayant de 2 à 6 atomes de carbone et alcynyle substitués ou non substitués ayant de 2 à 6 atomes de carbone, ou bien
R₅ et R₆ pris ensemble, ou R₇ et R₈ pris ensemble, forment un hétérocycle substitué ou non substitué à 5 à 7 chaînons contenant l'atome d'azote auquel ils sont rattachés, ou bien R₆ et R₇ pris ensemble, forment un hétérocycle substitué ou non substitué à 5 à 7 chaînons contenant le groupe uronium auquel ils sont rattachés, et
R₉ représente un hétérocycle substitué ou non substitué, à 5 à 7 chaînons, contenant l'atome d'azote auquel il est rattaché, et
X⁻ est un anion ;
dans laquelle ledit composé de marquage carboxylé est un composé luminescent contenant un fragment carboxylique.

2. Trousse selon la revendication 1, dans laquelle ledit composé luminescent est choisi parmi les coumarines contenant un fragment carboxylique, les fluorescéines contenant un fragment carboxylique et les rhodamines contenant un fragment carboxylique, les polyméthines contenant un fragment carboxylique y compris les cyanines, les mérocyanines, les oxazines, les thiazines, les phtalo- et naphtalo-cyanines, les diaza-indacènes, les 2,3-dihydrophtalazine-1,4-diones amino-substituées, les 2,3-dihydrobenzo[f]phtalazine-1,4-diones amino-substituées, les esters d'acridinium contenant un fragment carboxylique, les complexes luminescents de Ru(II), Os(II) et Ir(III) avec des diazines aromatiques contenant un fragment carboxylique, les complexes luminescents d'Eu(III) et Tb(III) contenant un fragment carboxylique, un dérivé de luminol à fonctionnalité carboxyle et ses analogues.

3. Trousse selon l'une quelconque des revendications précédentes, dans laquelle ladite amine tertiaire est choisie parmi la triéthylamine, la N,N-diisopropyléthylamine, la pyridine, la N,N-diméthylamino-pyridine, la 2,4,6-triméthylpyridine (collidine), la 4-méthylmorpholine, la 4-éthylmorpholine, la 4-morpholino-pyridine.

4. Trousse selon l'une quelconque des revendications précédentes, dans laquelle ledit premier composant et ledit deuxième composant sont formulés sous la forme soit de solides soit de solutions.

5. Trousse selon la revendication 4, dans laquelle ledit premier composant et ledit deuxième composant sont formulés sous la forme de solutions, le solvant étant choisi parmi le N,N-diméthylformamide, le diméthyl-sulfoxyde, le sulfolane, le N,N-diméthylacétamide, la N-méthylpyrrolidone, l'acétonitrile.

6. Trousse selon l'une quelconque des revendications précédentes, dans laquelle la quantité dudit deuxième composant est de 1/1 à 10/1, en rapport molaire par rapport audit composé de marquage carboxylé.

7. Trousse selon l'une quelconque des revendications précédentes, dans laquelle ledit sel de guanidinium de formule (a) est choisi parmi l'hexafluorophosphate d'O-(benzotriazol-1-yl)-N,N,N',N'-tétraméthyluronium (HBTU), le tétrafluoroborate d'O-(benzotriazol-1-yl)-N,N,N',N'-tétraméthyluronium (TBTU), l'hexafluorophosphate d'O-(7-azabenzotriazol-1-yl)-N,N,N',N'-tétraméthyluronium (HATU).

8. Trousse selon l'une quelconque des revendications 1 à 7, dans laquelle ledit sel d'uronium de formule (b) est choisi parmi le tétrafluoroborate de N,N,N',N'-tétraméthyl-O-(N-succinimidyl)uronium (TSTU), l'hexafluorophosphate de N,N,N',N'-tétraméthyl-O-(N-succinimidyl)uronium (HSTU), l'hexafluorophosphate de (1-cyano-2-éthoxy-2-oxoéthylidène-aminoxy)diméthylamino-morpholinecarbénium (COMU).

9. Utilisation d'un composé de marquage carboxylé, d'une amine tertiaire et d'un composé choisi parmi
i) un sel de guanidinium de formule (a) : 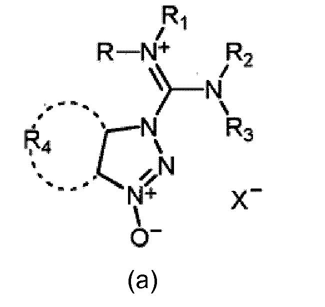 dans laquelle
R, R₁, R₂, R₃ sont indépendamment choisis parmi l'hydrogène et les radicaux alkyle substitués ou non substitués ayant de 1 à 6 atomes de carbone, alcényle substitués ou non substitués ayant de 2 à 6 atomes de carbone et alcynyle substitués ou non substitués ayant de 2 à 6 atomes de carbone, ou bien
R et R₁ pris ensemble, ou R₂ et R₃ pris ensemble, forment un hétérocycle substitué ou non substitué à 5 à 7 chaînons contenant l'atome d'azote auquel ils sont rattachés, ou bien
R et R₂ pris ensemble, forment un hétérocycle substitué ou non substitué à 5 à 7 chaînons contenant le groupe guanidinium auquel ils sont rattachés, et
R₄ représente un cycle aromatique ou hétéroaromatique substitué ou non substitué, à 6 à 10 chaînons, et
X⁻ est un anion ;
et
ii) un sel d'uronium de formule (b) : 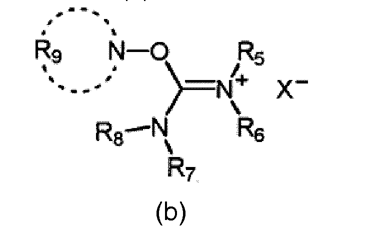 dans laquelle
R₅, R₆, R₇, R₈ sont indépendamment choisis parmi l'hydrogène et les radicaux alkyle substitués ou non substitués ayant de 1 à 6 atomes de carbone, alcényle substitués ou non substitués ayant de 2 à 6 atomes de carbone et alcynyle substitués ou non substitués ayant de 2 à 6 atomes de carbone, ou bien
R₅ et R₆ pris ensemble, ou R₇ et R₈ pris ensemble, forment un hétérocycle substitué ou non substitué à 5 à 7 chaînons contenant l'atome d'azote auquel ils sont rattachés, ou bien R₆ et R₇ pris ensemble forment un hétérocycle substitué ou non substitué à 5 à 7 chaînons contenant le groupe uronium auquel ils sont rattachés, et
R₉ représente un hétérocycle substitué ou non substitué, à 5 à 7 chaînons, contenant l'atome d'azote auquel il est rattaché, et
X⁻ est un anion ;
pour le marquage de biomolécules portant des groupes réactifs amino ou hydroxyle,
dans laquelle ledit composé de marquage carboxylé est un composé luminescent contenant un fragment carboxylique.

10. Utilisation selon la revendication 9, dans laquelle ledit composé luminescent est choisi parmi les coumarines contenant un fragment carboxylique, les fluorescéines contenant un fragment carboxylique et les rhodamines contenant un fragment carboxylique, les polyméthines contenant un fragment carboxylique y compris les cyanines, les mérocyanines, les oxazines, les thiazines, les phtalo- et naphtalo-cyanines, les diaza-indacènes, les 2,3-dihydrophtalazine-1,4-diones amino-substituées, les 2,3-dihydrobenzo[f]phtalazine-1,4-diones amino-substituées, les esters d'acridinium contenant un fragment carboxylique, les complexes luminescents de Ru(II), Os(II) et Ir(III) avec des diazines aromatiques contenant un fragment carboxylique, les complexes luminescents d'Eu(III) et Tb(III) contenant un fragment carboxylique, un dérivé de luminol à fonctionnalité carboxyle et ses analogues.

11. Utilisation selon l'une quelconque des revendications 9 et 10, dans laquelle ladite amine tertiaire est choisie parmi la triéthylamine, la N,N-diisopropyléthylamine, la pyridine, la N,N-diméthylamino-pyridine, la 2,4,6-triméthylpyridine (collidine), la 4-méthylmorpholine, la 4-éthylmorpholine, la 4-morpholino-pyridine.

12. Utilisation selon l'une quelconque des revendications 9 à 11, dans laquelle ledit sel de guanidinium de formule (a) est choisi parmi l'hexafluorophosphate d'O-(benzotriazol-1-yl)-N,N,N',N'-tétraméthyluronium (HBTU), le tétrafluoroborate d'O-(benzotriazol-1-yl)-N,N,N',N'-tétraméthyluronium (TBTU), l'hexafluorophosphate d'O-(7-azabenzotriazol-1-yl)-N,N,N',N'-tétraméthyluronium (HATU).

13. Utilisation selon l'une quelconque des revendications 9 à 11, dans laquelle ledit sel d'uronium de formule (b) est choisi parmi le tétrafluoroborate de N,N,N',N'-tétraméthyl-O-(N-succinimidyl)uronium (TSTU), l'hexafluorophosphate de N,N,N',N'-tétraméthyl-O-(N-succinimidyl)uronium (HSTU), l'hexafluorophosphate de (1-cyano-2-éthoxy-2-oxoéthylidène-aminoxy)diméthylamino-morpholinecarbénium (COMU).

14. Procédé pour marquer des biomolécules portant des groupes réactifs amino ou hydroxyle avec un composé de marquage carboxylé, comprenant les opérations consistant à :
i) disposer, dans des conditionnements séparés, d'un mélange du composé de marquage carboxylé et d'une amine tertiaire, et de l'un parmi :
- un sel de guanidinium de formule (a) : 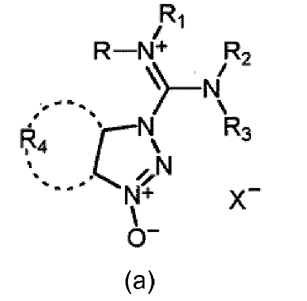 dans laquelle
R, R₁, R₂, R₃ sont indépendamment choisis parmi l'hydrogène et les radicaux alkyle substitués ou non substitués ayant de 1 à 6 atomes de carbone, alcényle substitués ou non substitués ayant de 2 à 6 atomes de carbone et alcynyle substitués ou non substitués ayant de 2 à 6 atomes de carbone, ou bien R et R₁ pris ensemble, ou R₂ et R₃ pris ensemble, forment un hétérocycle substitué ou non substitué à 5 à 7 chaînons contenant l'atome d'azote auquel ils sont rattachés, ou bien
R et R₂ pris ensemble, forment un hétérocycle substitué ou non substitué à 5 à 7 chaînons contenant le groupe guanidinium auquel ils sont rattachés, et
R₄ représente un cycle aromatique ou hétéroaromatique substitué ou non substitué, à 6 à 10 chaînons, et
X⁻ est un anion ;
et
- un sel d'uronium de formule (b) : 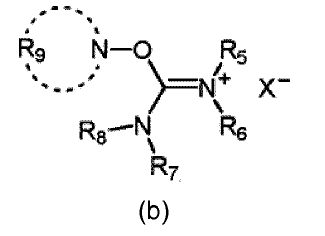 dans laquelle
R₅, R₆, R₇, R₈ sont indépendamment choisis parmi l'hydrogène et les radicaux alkyle substitués ou non substitués ayant de 1 à 6 atomes de carbone, alcényle substitués ou non substitués ayant de 2 à 6 atomes de carbone et alcynyle substitués ou non substitués ayant de 2 à 6 atomes de carbone, ou bien
R₅ et R₆ pris ensemble, ou R₇ et R₈ pris ensemble, forment un hétérocycle substitué ou non substitué à 5 à 7 chaînons contenant l'atome d'azote auquel ils sont rattachés, ou bien R₆ et R₇ pris ensemble forment un hétérocycle substitué ou non substitué à 5 à 7 chaînons contenant le groupe uronium auquel ils sont rattachés, et
R₉ représente un hétérocycle substitué ou non substitué, à 5 à 7 chaînons, contenant l'atome d'azote auquel il est rattaché, et
X⁻ est un anion ;
ii) mettre en contact le mélange et le sel de guanidinium ou le sel d'uronium, ce qui donne un composé de marquage carboxylé activé ;
iii) mettre en contact le composé de marquage carboxylé activé avec la biomolécule, ce qui donne une biomolécule marquée avec le composé de marquage carboxylé,
dans lequel lecomposé de marquage carboxylé est un composé luminescent contenant un fragment carboxylique.

15. Procédé selon la revendication 14 dans lequel ladite opération ii) est effectuée à une température comprise entre 0 et 60°C pendant une période de temps allant jusqu'à 60 minutes.

16. Procédé selon la revendication 14 ou la revendication 15, dans lequel ladite opération iii) est effectuée à une température comprise entre 4 et 50°C pendant une période de temps allant jusqu'à 24 heures.
